# EUROPÄISCHE PATENTANMELDUNG

(11) **EP 0 672 902 A1**
(43) Veröffentlichungstag der Anmeldung: **20.09.1995**
(21) Anmeldenummer: 94113934.7
(22) Anmeldetag: 06.09.1994
(51) Int. Cl.: G01N 21/90

(54) **Verfahren und Vorrichtungen zur Feststellung von Verunreinigungen in Flüssigkeiten**

(30) Priorität: 15.03.1994 DE 4408699
(71) Anmelder: MBM Kontroll-Systeme GmbH, D-84453 Mühldorf (DE)
(72) Erfinder:
(74) Vertreter: Seidel, Herta, Dipl.-Phys.

(57) **Zusammenfassung**

Ein Prüfverfahren zur Feststellung von als Verunreinigungen in Flüssigkeiten vorliegenden Festkörperpartikeln, bei dem sich die Flüssigkeiten in durchsichtigen verschließbaren, um ihre Längsachse drehbaren Behältern (Prüflingen) befinden, die mit Hilfe mindestens einer Reibvorrichtung um ihre Längsachse gedreht und an eine Prüfeinrichtung befördert werden, wo die sich in der Flüssigkeit befindenden, infolge der Drehung der Prüflinge in Drehung versetzte Partikel beobachtet werden, ist dadurch gekennzeichnet, daß die Drehung der Prüflinge dadurch bewirkt wird, daß sie mit ihrer Außenfläche in Kontakt mit mindestens einer Reibfläche gebracht und längs der oder den Reibfläche(n) zu der Prüfeinrichtung hin bewegt werden, wobei sich die Prüflinge infolge der Reibung selbsttätig um ihre Längsachse drehen und daß anschließend die Bewegung der Prüflinge im Bereich der Prüfeinrichtung angehalten und die Bahn der sich gegebenenfalls in der Flüssigkeit befindenden, sich weiterbewegenden Partikel beobachtet wird. Eine Vorrichtung zur Durchführung des Verfahrens umfaßt eine Reibvorrichtung mit mindestens einer Reibfläche und eine Fördervorrichtung, die dazu dient, die Prüflinge längs der oder den Reibfläche(n) entlang zu bewegen, um die Drehung der Prüflinge um ihre eigene Längsachse zu bewirken und um die Prüflinge von der Zuführungsvorrichtung zur Prüfeinrichtung zu transportieren, wo ihre Bewegung während des Beobachtungszeitraumes unterbrochen wird.

## Beschreibung

Die Erfindung betrifft ein Verfahren zur Feststellung von als Verunreinigungen in Flüssigkeiten vorliegenden Festkörperpartikeln, bei dem sich die Flüssigkeiten in durchsichtigen, verschließbaren, um ihre Längsachse drehbaren Behältern (Prüflingen) befinden, die mit Hilfe mindestens einer Reibvorrichtung um ihre Längsachse gedreht und an eine zumindest aus einer Lichtquelle und einem Sensor bestehende Prüfvorrichtung befördert werden, wo die sich in der Flüssigkeit befindenden, infolge der Drehung der Prüflinge in Drehung versetzten Partikel beobachtet werden.

Es ist bereits eine Vorrichtung der japanischen Firma ESAI bekannt geworden, mit der zur Feststellung, ob in Flüssigkeiten, die in durchsichtigen, verschließbaren und um ihre Längsachse drehbaren Behältern eingeschlossen sind, Luftblasen oder Festkörperpartikel vorliegen, eine sog. Bewegungsanalyse vorgenommen werden kann.

Bei dieser Vorrichtung befinden sich eine Mehrzahl von Prüflingen in einem Rundläufer, der eine kontinuierliche Drehbewegung ausführt, und werden von diesem auf einer Kreisbahn von einer Zuführungsstation zu einer Prüfstation und schließlich zu einer Ausgabestation bewegt. Auf einem ersten Abschnitt dieser Kreisbahn erfahren die zwischen ihrer Deckfläche und Bodenfläche in Haltevorrichtungen eingeklemmten Prüflinge mittels eines Reibbandes, das eine Rotation der Haltevorrichtungen bewirkt, eine Rotation um ihre Längsachse. Hierdurch wird auch die in den Behältern eingeschlossene Flüssigkeit in Drehbewegung versetzt. In einem zweiten Abschnitt der Kreisbahn werden die Prüflinge nach Beendigung ihrer Rotation mit einer Prüfeinrichtung beobachtet, die mit einer mit dem Rundläufer übereinstimmenden Winkelgeschwindigkeit innerhalb eines vorgegebenen Bereiches hin und her bewegt wird. Infolge der Synchronbewegung von Prüfeinrichtung und Rundläufer ist während der Beobachtungszeit zwischen dem Prüfling und der Prüfeinrichtung die Relativgeschwindigkeit null. Nur unter der Voraussetzung dieser Synchronbewegung von Prüfling und Prüfeinrichtung können bei dieser Bildanalyse die sich infolge der vorangegangenen Rotation der Prüflinge in der Flüssigkeit bewegenden Luftblasen oder Partikel festgestellt und aufgrund des Verlaufs ihrer Bewegungsbahn unterschieden werden. Die sich in konzentrischen Kreisen um die Längsmittelachse der Prüflinge drehenden Partikel werden nach Beendigung des Rotationsantriebs absinken und eine nach unten gerichtete spiralförmige Bewegung ausführen, während die Luftblasen in kreisförmigen Bahnen nach oben aufsteigen. Diese unterschiedlichen Bewegungsrichtungen erlauben daher eine klare Unterscheidung zwischen dem Vorhandensein von Partikeln oder von Luftblasen in einer Flüssigkeit. Da infolge der Synchronbewegung von Prüfling und Prüfeinrichtung bei der Beobachtung keine Relativgeschwindigkeit zwischen beiden Vorrichtungen vorliegt, wirkt sich an der Behälteraußenwand anhaftender Schmutz bei der Analyse nicht nachteilig aus, da diese Verschmutzung bei der Beobachtung keine Bewegung ausführt.

Dieses bekannte Verfahren zur Bewegungsanalyse zur Unterscheidung von in Flüssigkeiten vorhandenen Festkörperpartikeln und Luftblasen hat aber den Nachteil, daß die feinmechanischen Erfordernisse für eine Justierung der Prüflinge sehr hoch sind und auch die Gewährleistung einer Synchronbewegung von Prüfling und Prüfeinrichtung so große technische Probleme aufwirft, daß der Erfolg der bekannten Methode in Frage gestellt wird.

Aufgabe der vorliegenden Erfindung ist es daher, ein Verfahren anzugeben, bei dem die bei dem bekannten Verfahren kritischen Verfahrensabschnitte umgangen werden und mit geringerem mechanischem Aufwand und dadurch auch geringeren Kosten eine empfindlichere Bildanalyse ermöglicht wird.

Erfindungsgemäß wird dies durch ein Verfahren erreicht, das dadurch gekennzeichnet ist, daß die Drehung der Prüflinge dadurch bewirkt wird, daß sie mit ihrer Außenfläche in Kontakt mit mindestens einer Reibfläche gebrachtund längs der oder den Reibflächen zu der Prüfeinrichtung hin bewegt werden, wobei sich die Prüflinge infolge der Reibung selbsttätig um ihre Längsachse drehen und daß anschließend die Bewegung der Prüflinge im Bereich der Prüfeinrichtung angehalten und die Bahn der sich gegebenenfalls in der Flüssigkeit befindenden, sich weiterbewegenden Partikel beobachtet wird.

Bei dem erfindungsgemäßen Verfahren führen die Prüflinge eine intermittierende Bewegung aus, sie werden nämlich zur Prüfeinrichtung hin bewegt und stehen aber während des Beobachtungszeitraumes, ebenso wie auch die Prüfeinrichtung, still. Das heißt, es bewegen sich bei der Beobachtung lediglich die evtl. in der Flüssigkeit vorhandenen Festkörperpartikel und die Luftblasen. Hierdurch kann die Bildanalyse wesentlich empfindlicher eingestellt werden, so daß bei diesem Verfahren noch viel kleinere Partikel in einer Flüssigkeit erkannt werden können.

Ferner ist von Vorteil, daß die Prüfeinrichtung keinen hohen Beschleunigungen ausgesetzt wird, was eine beachtliche Erleichterung für deren Justierung mit sich bringt.

Weitere vorteilhafte Ausbildungen des Verfahrens ergeben sich aus den in den Unteransprüchen aufgeführten Merkmalen.

Für die Durchführung des erfindungsgemäßen Verfahrens werden nachstehend vier Vorrichtungen beschrieben, die auf dem erfindungsgemäßen Prinzip einer intermittierenden Bewegung der Prüflinge beruhen. Jedoch ist der Schutzumfang nicht auf die genannten Vorrichtungen beschränkt, vielmehr liegt es im Bereich des Durchschnittsfachmanns, aufgrund der gegebenen Anregungen Abwandlungen der speziellen Ausführungsformen zu konzipieren, die innerhalb des offenbarten Erfindungsgedankens liegen.

Die erfindungsgemäßen Vorrichtungen zur Durchführung des Verfahrens umfassen mindestens eine Zuführungsvorrichtung für die Zuführung der Prüflinge an eine Reibvorrichtung, die die Drehung der Prüflinge um ihre Längsachse bewirkt und mindestens eine Prüfeinrichtung für die Feststellung der in der zu prüfenden Flüssigkeit enthaltenen Festkörperpartikel und mindestens eine Ausgabevorrichtung. Ihnen ist gemeinsam, daß sie dadurch gekennzeichnet sind, daß die Reibvorrichtung mindesten eine Reibfläche und eine Fördervorrichtung umfaßt, die dazu dient, die Prüflinge längs der oder den Reibflächen entlang zu bewegen, um die Drehung der Prüflinge um ihre eigene Längsachse zu bewirken und um die Prüflinge von der Zuführungsvorrichtung zur Prüfeinrichtung zu transportieren, wo ihre Bewegung während des Beobachtungszeitraumes unterbrochen ist.

Gemäß einer Ausbildungsform ist die Fördervorrichtung um eine parallel zur Längsachse der Prüflinge verlaufende Drehachse drehbar und umfaßt eine Mehrzahl von radial nach außen verlaufenden Armen, an deren Außenrand Halteorgane in Form von Rollen- oder Gleitlagern vorgesehen sind, für die Bewegung der von der Zuführungsvorrichtung her zugeführten Prüflinge längs der im Umfangsbereich der Fördervorrichtung zwischen der Zuführungsvorrichtung und der Prüfeinrichtung angeordneten Reibfläche, wobei vorzugsweise die Fördervorrichtung mit einer in bestimmbarem Zeittakt schaltbaren Bremsvorrichtung ausgestattet ist, die ermöglicht, daß die Drehbewegung der Fördervorrichtung intermittierend erfolgt und dann zu unterbrechen ist, wenn ein Prüfling nach erfolgter Drehung um seine eigene Längsachse in den Bereich einer der außerhalb des Umfanges der Fördervorrichtung stationär angeordneten Prüfeinrichtungen gelangt ist.

Gemäß einer weiteren Ausbildungsform umfaßt die Reibvorrichtung einen Förderkanal, dessen eine Längsseitenwand durch die Reibfläche gebildet ist, deren Reibungskoeffizient groß in Bezug auf das Material der Prüflinge ist und durch die die Prüflinge bei ihrer Bewegung durch den Förderkanal zur Rotation gebracht werden und die Fördervorrichtung weist einen hin- und herbwegbaren Stössel auf, der die Prüflinge zumindest bis zum Förderkanal transportiert, so daß die Prüflinge nach Durchlaufen des Förderkanales in Ruheposition im Bereich der Prüfeinrichtung beobachtbar und beurteilbar sind, bevor sie zur Ausgabevorrichtung gelangen. Vorzugsweise umfaßt die Fördervorrichtung zum Transport der Prüflinge durch den Kanal ein umlaufendes Transportband, das für die Führung der Prüflinge mit Rollen oder dergleichen versehen ist, oder der hin- und herbewegbare Stössel kann zur Beförderung der Prüflinge durch den Kanal dienen. Hierzu ist er an seiner Stirnfläche mit einer oder mit mehreren Rollen versehen und die Reibfläche vorzugsweise in Form eines umlaufenden Reibbandes ausgebildet.

Die Ausbildungen dieser Vorrichtungen sind nachstehend näher erläutert. Weitere Merkmale der erfindungsgemäßen Vorrichtungen ergeben sich aus den Unteransprüchen sowie aus der nachstehenden Beschreibung von Ausführungsbeispielen anhand der Zeichnung. Hierin zeigen:
- Fig. 1:: eine Draufsicht auf eine Vorrichtung zur Feststellung von Verunreinigungen in Flüssigkeiten in schematischer Darstellung
- Fig. 2:: eine abgebrochene Darstellung einer Seitenansicht eines Halteorgans für Prüflinge in Form von Einweginjektionspritzen.
- Fig. 3:: eine abgebrochene Darstellung einer Seitenansicht eines weiteren Halteorgans für beidseitig verschließbare Ampullen
- Fig. 4a:: eine Seitenansicht des Transportsterns mit einem Halteorgan
- Fig. 4b:: einen Schnitt durch den Transportstern mit Halteorgan
- Fig. 5:: eine Draufsicht auf eine weitere Vorrichtung für die Durchführung einer Bewegungsanalyse in schematischer Darstellung und
- Fig. 6:: eine weitere Vorrichtung für die Durchführung einer Bewegungsanalyse in schematischer Darstellung
- Fig. 7:: eine weitere Vorrichtung für die Durchführun einer Bewegungsanalyse in schematischer Darstellung.

In den Figuren sind sich entsprechende Teile mit übereinstimmenden Bezugsziffern gekennzeichnet.

Über eine nur schematisch angedeutete herkömmliche Zuführungsvorrichtung 1 gelangen die mit der zu untersuchenden Flüssigkeit gefüllten , verschlossenen Prüflinge 2a,b,c..n, nämlich Ampullen, Vials, Einwegspritzen, Infusionsbehälter und dgl. in den Bereich von zwei benachbart zueinander, am freien Ende eines Armes 3 eines Transportsterns 4 nach oben über den Arm 3 hinaus ragenden, drehbar im Arm 3 gelagerten Rollen 5a,b. Der Transportstern 4 ist umgeben von einem Gehäuse 6. Längs der Umlaufbahn der Rollen 5a,b ist eine Führungswand 7 angeordnet. Längs der Innenfläche der Führungswand 7 verläuft ein Silikonschlauch 9. Sobald der Transportstern 4 mit Hilfe eines Antriebsmotors, der vorzugsweise als Servomotor ausgebildet ist, um seine Drehachse 10 gedreht wird, wird der sich zwischen den beiden Rollen 5a,b befindende Prüfling 2a mitgenommen und gegen den elastischen Schlauch 9 gedrückt. Durch die Reibung wird der Prüfling 2a so gut gehalten, daß keine Bodenauflage für den Prüfling erforderlich ist. Dies hat den Vorteil, daß, falls der Bedarf hierfür vorliegt, auch der Boden des Prüflings inspiziert werden kann. Da die Reibung zwischen dem Schlauch 9 und der Außenwand des Prüflings 2a größer ist als die rollende Reibung zwischen den Rollen 5a,b und dem Prüfling 2a, rotiert der Prüfling 2a bei seiner Bewegung von der Zuführungsstation 1 zum Auslaufbereich 8 innerhalb der Führungswand 7 um seine eigene Längsachse. Der Prüfling 2a,b...n ist nämlich zwischen den Rollen 5a,b drehbar gelagert und wird durch die Bewegung entlang des Silikonschlauches 9 in Drehung versetzt. Auch die im Prüfling 2a eingeschlossene Flüssigkeit beginnt dabei zu rotieren.

Sobald der Arm 3 des Transportsterns 4 einen Winkelbereich von ca. 180° zurückgelegt hat, wird die Bewegung des Transportsterns 4 durch den Motor bis zu seinem Stillstand abgebremst.

Die beiden Rollen 5a,b und der von ihnen gehaltene Prüfling 2a befinden sich dann gerade an der Stelle, wo sich die Prüfeinrichtung 11 befindet. Die Prüfeinrichtung 11 umfaßt eine Beleuchtungseinrichtung 12 und einen Sensor 13. Bei der Bildanalyse wird nun festgestellt, ob in der sich im Prüfling 2a nach dessen Stillstand noch weiterdrehenden Flüssigkeit Luftblasen oder Festkörperpartikel vorhanden sind. Dabei unterscheiden sich vorhandene Luftblasen in ihrer Bewegungsbahn von den Festkörperpartikeln. Luftblasen steigen in einer spiralförmigen Bewegung in dem Prüfling nach oben, während Festkörperpartikel aufgrund ihres Gewichts in einer spiralförmigen Bahn nach unten sinken. An der Außenwand des Prüflings vorhandene Verschmutzungen werden nicht als Fehler erkannt, da sie sich nicht bewegen, denn der Prüfling ruht während des Prüfvorgangs. Nach Beendigung der Bildanalyse dreht sich der Transportstern 4 weiter, der zwischen den Rollen 5a,b gehaltene Prüfling 2a gelangt in die Ausgabevorrichtung 8. Während sich jedoch der Arm 3 mit dem Prüfling 2a im Bereich der Prüfeinrichtung 11 befindet, gelangt aus der Zuführungsvorrichtung 1 ein weiterer Prüfling 2b zwischen das am diametral vom Dreharm 3 gegenüberliegende Ende des Dreharmes 14 angeordnete Rollenpaar 15a,b und wird, während sich der Dreharm 3 in seine Ausgangsposition bewegt, entsprechend wie vorher der Prüfling 2a, längs der Führungswand 7, unter Rotation um seine Längsachse, an die Prüfeinrichtung 11 bewegt.

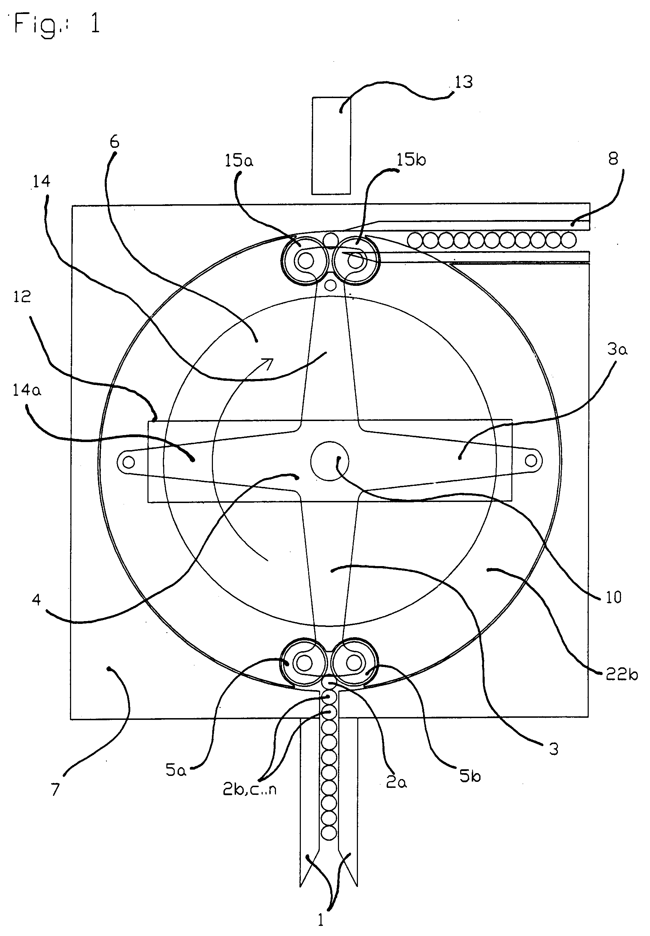

In der Fig. 1 weist der Transportstern 4 zwei Armkreuze 3, 14 und 3a, 14a auf. Während nur zwei sich gegenüberliegende Rollenpaare 5a,b und 15a,b am Armkreuz 3 bzw. 14 dargestellt sind, können aber auch an den beiden anderen Armen 3a, 14a des Armkreuzes Rollenpaare angeordnet sein. In diesem Falle ist natürlich für eine weitere Zuführungsvorrichtung und eine weitere Ausgabevorrichtung zu sorgen. Ferner ist eine weitere Prüfeinrichtung vorzusehen. Der Drehwinkel des Transportsterns 4 beträgt, bis der Prüfling zur Prüfeinrichtung kommt, dann nur noch 90°.

Es ist auch denkbar, zur Erhöhung der Inspektionsrate eine noch größere Anzahl von Zuführungs- und Ausgabevorrichtungen mit einer entsprechenden Anzahl von Prüfeinrichtungen vorzusehen. Der Transportstern 4, der mit der entsprechenden Anzahl von Armen ausgestattet sein muß, hat dann eine von der für eine Bildanalyse erforderlichen Zeit bestimmte intermittierende Bewegung zu durchlaufen.

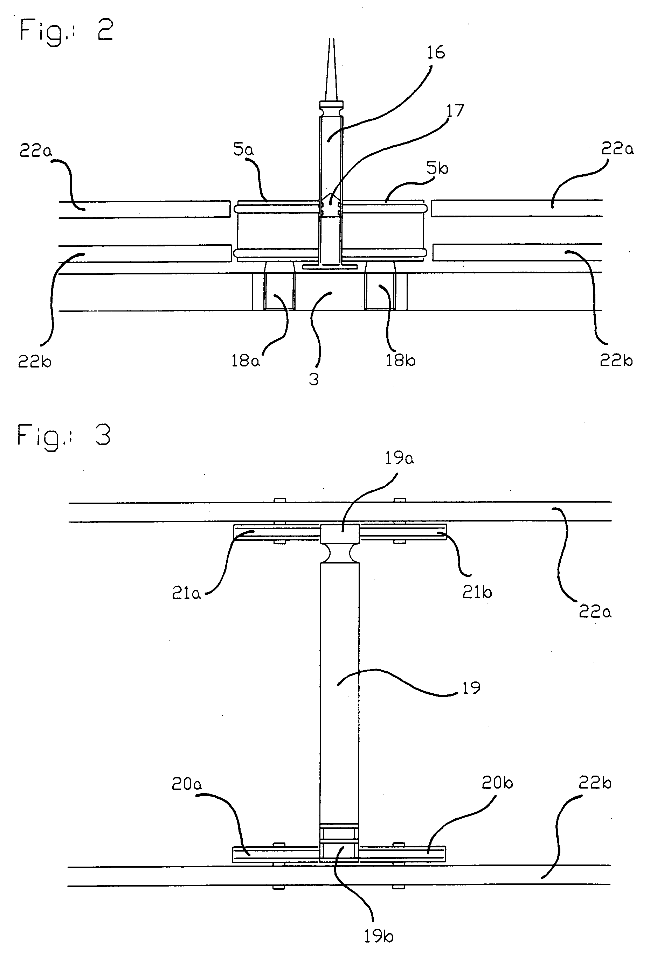

In Fig. 2 ist eine Seitenansicht einer zwischen zwei Rollen 5a,b gehaltenen Einweginjektionsspritze 16 dargestellt. Der undurchsichtige Verschlußstopfen 17 der Spritze 16 befindet sich zwischen den beiden Rollen 5a,b, während die durchsichtige Wandung der Spritze 16 frei nach oben ragt und ihr gesamter Flüssigkeitsbereich vollständig sichtbar ist, so daß er von einer Prüfeinrichtung 11 analysiert werden kann. In diesem Beispiel ist längs der Führungswand 7 ein Silikonschlauch 9 angeordnet. Ferner ist die Welle 18a,b der Rollen 5a,b im Arm 3 des Transportarmes 4 zu erkennen.

In Fig. 3 ist ein an beiden Enden mit Stopfen 19a,b verschlossener Prüfling 19 dargestellt. Für Prüflinge,deren Flüssigkeitsbereich länger ist, wie hier bei dem Prüfling 19, sind die Rollen 20a,b und 21a,b in einem der Länge des Prüflings 19 entsprechenden Abstand in unterteilten Führungsscheiben 22a,b gelagert. Auch hierbei wird wiederum erreicht, daß der gesamte durchsichtige Bereich des Prüflings 19 auf das Vorhandensein von Festkörperpartikeln bzw.Luftblasen inspiziert werden kann.

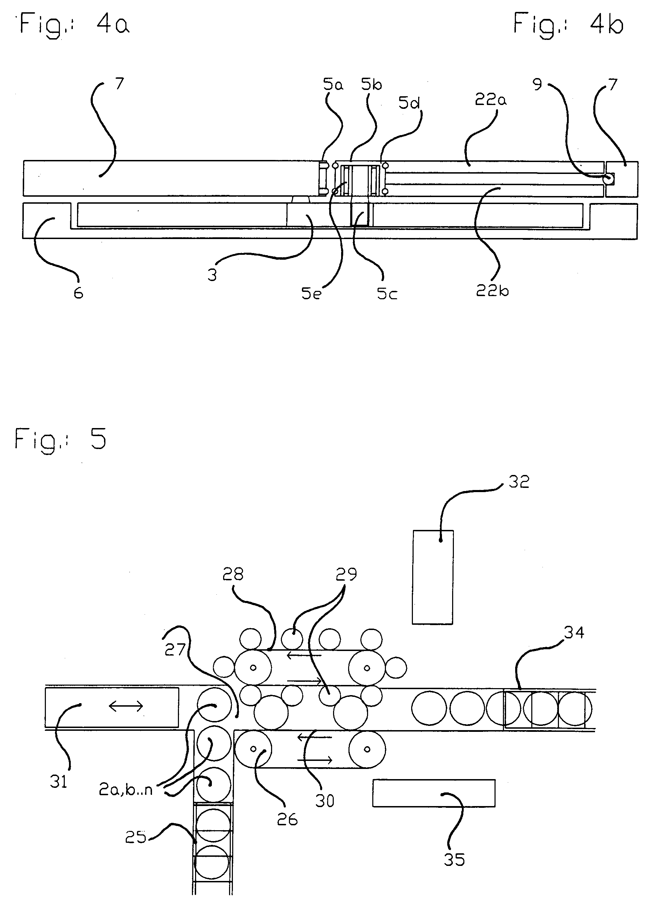

In Fig. 4a ist eine Seitenansicht des Eingangsbereiches A des Transportsterns 4 dargestellt. In Fig. 4b ist ein Schnittbild durch den Eingangsbereich 1 des Transportsterns 4 gezeigt.

Im Arm 3, der von einem Gehäuse 6 umgeben ist, sind die Rollen 5a bzw. 5b gelagert. Die Rolle 5a ist von der Führungswand 7 fast vollständig eingefaßt. Aus Fig. 4b ist zu erkennen, daß längs der Innenfläche der Führungswand 7 der Silikonschlauch 9 angeordnet ist. Die Rolle 5b umfaßt eine im Arm 3 gelagerte Achse 5c, eine zylindrische Kappe 5d ist auf einem Nadellager 5e drehbar gelagert.

Die vorstehend beschriebenen Konstruktionsdetails sind jedoch lediglich als mögliche Ausführungsformen zu bewerten. Dem Durchschnittsfachmann ist es jedoch möglich, technisch äquivalente Konstruktionslösungen anzugeben und diese, den unterschiedlichen Behälterformen anzupassen. Dabei kann die jeweils kostengünstigste Vorrichtung ausgewählt werden.

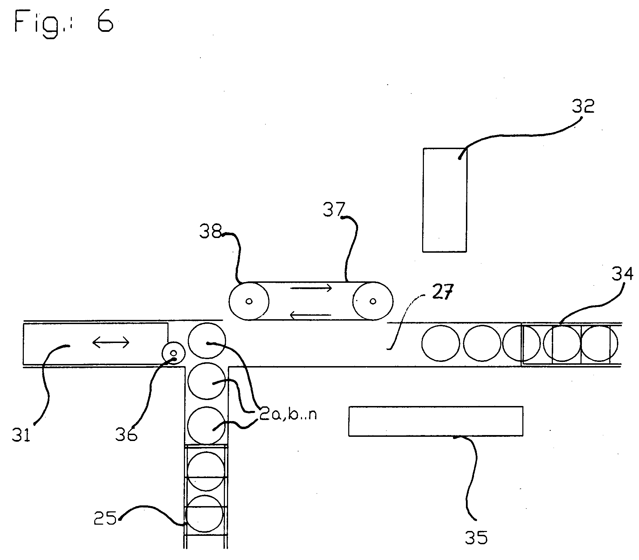

Die in Fig. 5 gezeigte weitere Ausbildungsform einer Vorrichtung zur Durchführung einer Bewegungsanalyse von in Flüssigkeiten enthaltenen Festkörperpartikeln ist ebenfalls nur schematisch dargestellt, da Einzelheiten einer konstruktiven Lösung jedem Fachmann geläufig sein dürften.

Eine Zuführungseinrichtung 25 transportiert in herkömmlicher Weise die aufrecht stehenden Prüflinge 2a,b..n zu der Reibvorrichtung 26. Die Reibvorrichtung 26 in Form eines Förderkanals 27 umfaßt ein umlaufendes Transportband 28 mit einer glatten Oberfläche und mit in den Förderkanal 27 hineinragenden Vorsprüngen 29 oder Rollen. Die gegenüberliegende Wandfläche des Förderkanals 27 ist mit einem elastischen Reibbelag 30 belegt, der auch in Form eines in Gegenrichtung umlaufenden Reibbandes ausgeführt sein kann. Von der Zuführungsvorrichtung 25 angelieferte Prüflinge 2a,b..n werden mittels eines hin- und herbewegbaren Stössels 31 in den Anfangsbereich des Förderkanals 27 bewegt und dort mit Hilfe eines der Vorsprünge 29 oder Rollen erfaßt und von diesen durch den Förderkanal 27 geschleust. Dabei rollen die Prüflinge 2a,b..n, rotierend um ihre eigene Längsachse, längs des Reibbelags 30 durch den Förderkanal 27 hindurch und treten im Bereich der Prüfeinrichtung 32 aus dem Kanal 27 heraus. Hierbei wird ihre Bewegung unterbrochen, sie stehen jetzt still im Meßbereich der Prüfeinrichtung 32, während die Flüssigkeit in ihrem Inneren noch weiter rotiert und eine Bewegungsanalyse auf in der Flüssigkeit vorhandene Verunreinigungen ermöglicht. Sobald diese Prüfung abgeschlossen ist, wird der Prüfling von dem bzw. den nachfolgenden Prüflingen zur Ausgabevorrichtung 34 geschoben, die ebenfalls in herkömmlicher Weise konstruiert ist. Die für die Bewegungsanalyse benötigte Zeit bestimmt die Anzahl der gleichzeitig in dem Förderkanal 27 transportierbaren Prüflinge 2a,b..n. Jedem Fachmann ist es möglich, die Länge des Förderkanals 27 und die Anzahl der gleichzeitig im Förderkanal 27 befindlichen zur Prüfeinrichtung 32 hin transportierbaren Prüflinge 2a,b..n in Abhängigkeit von der Dauer der Prüfung eines einzigen Prüflings und den jeweils vorliegenden Durchmessern der Prüflinge anzupassen. Da aber durch die Wahl einer stationären Prüfeinrichtung die Durchführung der Prüfung gegenüber den herkömmlich bekannten Verfahren stark vereinfacht ist, kann die Anzahl der Prüfungen pro Zeiteinheit gegenüber der bekannten Vorrichtung stark erhöht werden.

Eine weitere Ausbildung der Vorrichtung zur Feststellung von Verunreinigungen in Flüssigkeiten ist in Fig. 6 gezeigt. Hier ist das umlaufende Transportband 28 durch eine um Walzen 38 umlaufende Reibfläche oder Reibband 37 ersetzt.

Bei dieser Ausführungsform muß allerdings der Stössel 31 mit einer Stirnfläche ausgebildet sein, die aus mindestens einer Leerlaufrolle 36 oder besonders glatten Gleitflächen (nicht dargestellt) besteht. Dieser Stössel 31 hat dann die Aufgabe, die Prüflinge 2a,b..n durch den Kanal 27 zur Meßeinrichtung 32 zu transportieren. Aufgrund der geringen Reibung der an seiner Stirnfläche vorgesehenen Rollen oder Gleitfläche und der Reibfläche 37 wird sich der Prüfling während seiner Bahn durch den Kanal 27 ebenfalls selbsttätig um seine eigene Längsachse drehen.

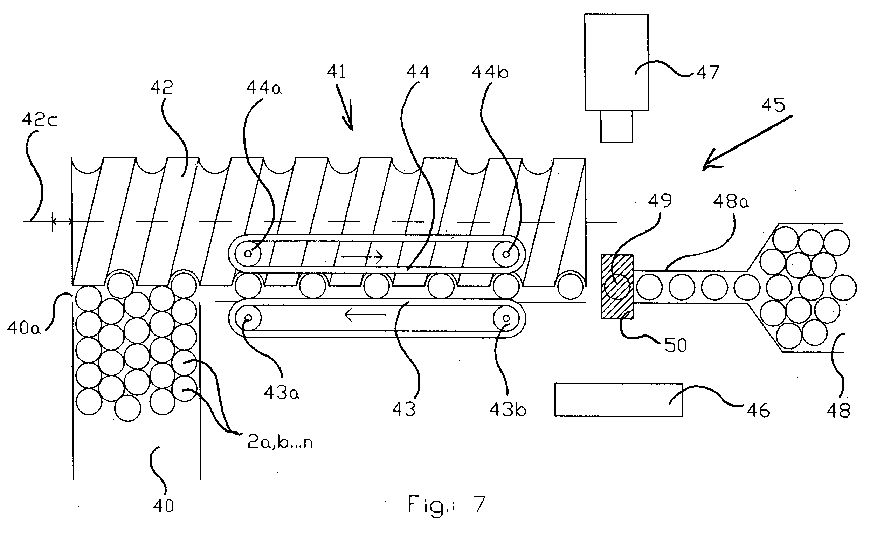

Die in Fig. 7 gezeigte Vorrichtung zur Durchführung der Bewegungsanalyse von in drehbaren Behältern (Prüflingen) 2a..n befindlichen, verunreinigten Flüssigkeiten umfaßt ein Einlaufmagazin 40, eine Reib- und Fördervorrichtung 41, die ihrerseits eine Förderschnecke 42 und eine Rotationseinrichtung mit umlaufenden Reibbändern 43 und 44 umfaßt, ferner eine Prüfeinrichtung 45 mit einer Beleuchtungseinrichtung 46 und einer Kamera 47 sowie ein Auslaufmagazin 48. Über der Prüfposition 49 unmittelbar vor dem Eingang des Auslaufmagazins 48 ist eine Bremseinrichtung 50 vorgesehen, die die Aufgabe hat, die von der Reib- und Fördervorrichtung 41 an die Prüfeinrichtung 45 angelieferten, in Drehbewegung befindlichen Prüflinge 2a..n bis zum Stillstand abzubremsen. Die Förderschnecke 42 ist in ihrer Längsachse hin- und herverschiebbar gelagert und wird mittels eines Elektromotors gedreht und mittels Feder und Nocken hin- und herbewegt. Der Takt der Hin- und Herbewegung ergibt sich aus der Dauer der Prüfung eines in der Prüfeinrichtung 45 befindlichen Prüflinges 2a..n.

Die Funktionsweise der Vorrichtung nach Fig. 7 ist folgende: Im Einlaufmagazin 40 befindet sich eine Vielzahl von zu untersuchenden Prüflingen 2a,b...n, deren Längsachsen aufrechtstehen und die parallel zueinander ausgerichtet sind. Quer zur Ausgabeöffnung 40a des Einlaufmagazins 40 verläuft die Längsachse 42c einer Förderschnecke 42, deren Windungen 42a einen Abstand voneinander haben, der etwa dem Durchmesser der Prüflinge 2a..n entspricht und deren Windungstäler 42b eine Tiefe haben, die etwa dem Radius der Prüflinge 2a..n entspricht. Mittels eines nicht dargestellten Motors wird die Förderschnecke 42 um ihre Längsachse 42c gedreht und dabei mittels Feder und Nocken gleichzeitig zu einer Hin- und Herbewegung in Richtung ihrer Längsachse 42c veranlaßt. Dabei gelangen Prüflinge 2a..n in die Windungstäler 42b und werden von diesen in Richtung der Längsachse 42c der Förderschnecke 42 mitgenommen und gelangen schließlich zu zwei parallel zur Längsachse 42c angeordneten Reibbändern 43 und 44, die als Rotationseinrichtung dienen. Das Reibband 43 wird durch eine Rolle 43a im Gegenuhrzeigersinn angetrieben und durch die Leerlaufrolle 43b zu einer Reibstrecke parallel zur Längsachse 42c der Förderschnecke 42 gespannt. Das Reibband 44 wird durch eine Rolle 44a zu einem Umlauf im Uhrzeigersinn angetrieben und mit Hilfe der Leerlaufrolle 44b ebenfalls zu einer Reibstrecke gespannt, die parallel zu derjenigen des Reibbandes 43 verläuft. Die Bahngeschwindigkeiten der beiden Reibbänder 43 und 44 sind so gewählt, daß die Transportgeschwindigkeit des Reibbandes 44 um einen Betrag größer ist als die Transportgeschwindigkeit des Reibbandes 43, der genau der Transportgeschwindigkeit der Förderschnecke 42 entspricht. Hierdurch werden die zwischen die Reibstrecken beider Reibbänder 43 und 44 eingebrachten Prüflinge in Rotation um ihre Längsachse versetzt und gelangen schließlich an die Stirnseite der Förderschnecke 42, die benachbart zur Prüfposition 49 für die Prüflinge 2a..n liegt.

Infolge der Hin-und Herbewegung der Förderschnecke 42, deren Hub genau dem Abstand der Prüfposition 49 von der Stirnfläche der Förderschnecke 42 entspricht, wird der im Stirnbereich angelangte Prüfling 2a..n auf die Prüfposition 49 geschoben, das heißt, er wird aus dem Wirkungsbereich der Förderschnecke 42 entfernt und gleichzeitig durch Absenken einer durch das schraffierte Rechteck schematisch dargestellten Bremsvorrichtung 50 an einer weiteren Drehung gehindert. Im ruhenden Behälter des Prüflings 2a..n rotiert der Flüssigkeitsinhalt aber weiter, so daß die in der Flüssigkeit enthaltene Verunreinigung oder Luftblasen nach dem vorstehend erläuterten Prüfprinzip einer Bewegungsanalyse, durchleuchtet von der Beleuchtungseinrichtung 46, mittels der Kamera 47 festgestellt werden können. Nach beendigter Prüfung wird der gerade geprüfte Prüfling 2a..n durch einen nachfolgenden, von der Förderschnecke 42 angelieferten Prüfling 2a..n in den Eingangskanal 48a des Auslaufmagazins 49 weitergeschoben und kann anschließend entsprechend dem Prüfergebnis sortiert werden.

Der Vorteil dieser Vorrichtung liegt in der kontinuierlichen Arbeitsweise der Förderschnecke, deren Fördergeschwindigkeit allein von der Dauer der Prüfung eines Prüflings abhängt, die aber erfahrungsgemäß sehr kurz ist.

Bei allen vorstehend beschriebenen Ausführungsformen der Vorrichtungen für die Durchführung einer Bewegungsanalyse ist eine Beobachtungseinrichtung gezeigt, durch die eine Hellfeldbeleuchtung des Prüflings erzielt wird. Die Beobachtung kann aber auch als Dunkelfeldbeleuchtung ausgebildet sein, indem sich die Beleuchung oberhalb des Prüflings befindet.

## Patentansprüche

1. Prüfverfahren zur Feststellung von als Verunreinigungen in Flüssigkeiten vorliegenden Festkörperpartikeln, bei dem sich die Flüssigkeiten in durchsichtigen verschließbaren, um ihre Längsachse drehbaren Behältern (Prüflingen) befinden, die mit Hilfe mindestens einer Reibvorrichtung um ihre Längsachse gedreht und an eine zumindest aus einer Lichtquelle und einem Sensor bestehenden Prüfeinrichtung befördert werden, wo die sich in der Flüssigkeit befindenden, infolge der Drehung der Prüflinge in Drehung versetzte Partikel beobachtet werden, **dadurch gekennzeichnet,** daß die Drehung der Prüflinge dadurch bewirkt wird, daß sie mit ihrer Außenfläche in Kontakt mit mindestens einer Reibfläche gebracht und längs der oder den Reibflächen zu der Prüfeinrichtung hin bewegt werden, wobei sich die Prüflinge infolge der Reibung selbsttätig um ihre Längsachse drehen und daß anschließend die Bewegung der Prüflinge im Bereich der Prüfeinrichtung angehalten und die Bahn der sich gegebenenfalls in der Flüssigkeit befindenden, sich weiterbewegenden Partikel beobachtet wird.

2. Prüverfahren nach Anspruch 1, **dadurch gekennzeicnet**, daß die Bewegung des Prüflings längs der oder den Reibflächen nach erzielter Drehung um seine Längsachse unterbrochen wird (intermittierende Bewegung des Prüflings) und die Überprüfung der sich in der Flüssigkeit weiterbewegenden Partikel bei stillstehendem Prüfling mit Hilfe einer stationär angeordneten Prüfvorrichtung durchgeführt wird.

3. Prüfverfahren nach Anspruch 1 oder 2, **dadurch gekennzeichnet,** daß der Prüfling während seiner Bewegung längs der oder den Reibflächen mit Hilfe einer Rollen- oder Gleitführungsvorrichtung drehbar gehalten und bewegt wird, deren Reibungskoeffizient klein gegenüber dem Reibungskoeffizient zwischen Reibfläche und Prüfling gewählt wird.

4. Prüfverfahren nach einem der Ansprüche 1 bis 3, **dadurch gekennzeichnet,** daß die Anzahl der gleichzeitig längs der Reibfläche bewegbaren Prüflinge durch die Arbeitsgeschwindigkeit der Prüfeinrichtung bestimmt wird.

5. Prüfverfahren nach einem der vorhergehenden Ansprüche, **dadurch gekennzeichnet**, daß erst nach erfolgter Prüfung eines Prüflings mit der Bewegung des nachfolgenden Prüflings längs der oder den Reibflächen begonnen wird.

6. Prüfverfahren nach einem der vorhergehenden Ansprüche, **dadurch gekennzeichnet**, daß die Zuführung aufeinanderfolgender Prüflinge an die Prüfstation kontinuierlich erfolgt und die Bewegung jedes Prüflings in der Prüfstation vor Beginn der Prüfung abgebremst wird.

7. Vorrichtung zur Durchführung des Verfahrens nach einem der Ansprüche 1 bis 6 mit mindestens einer Zuführungsvorrichtung für die Zuführung der Prüflinge an mindestens eine Reibvorrichtung, die die Drehung der Prüflinge um ihre Längsachse bewirkt, mit mindestens einer Prüfeinrichtung für die Feststellung der in der zu prüfenden Flüssigkeit enthaltenen Festkörperpartikel und mit mindestens einer Ausgabevorrichtung, **dadurch gekennzeichnet**, daß die Reibvorrichtung(en) mindestens eine Reibfläche (9, 30,43,44) und eine Fördervorrichtung (4, 28, 31,42) umfassen, die dazu dient, die Prüflinge (2a,b ...n) längs der oder den Reibflächen (9, 30,43,44) entlangzubewegen, um die Drehung der Prüflinge um ihre eigene Längsachse zu bewirken und um die Prüflinge von der Zuführungsvorrichtung (1, 25,40) zur Prüfeinrichtung (11,32,45) zu transportieren, wo ihre Bewegung während des Beobachtungszeitraumes unterbrochen ist.

8. Vorrichtung nach Anspruch 7, **dadurch gekennzeichnet**, daß die Fördervorrichtung (4) um eine parallel zur Längsachse der Prüflinge (2a, b...n) verlaufende Drehachse (10 drehbar ist und eine Mehrzahl von radial nach außen verlaufenden Armen (3, 4, 3a, 4a) umfaßt, an deren Außenrand Halteorgane (5a,b, 15a,b) in Form von Rollen- oder Gleitlagern vorgesehen sind für die Bewegung der von der Zuführungsvorrichtung (1) her zugeführten Prüflinge längs der im Umfangsbereich (7) der Fördervorrichtung (4) zwischen der Zuführungsvorrichtung (1) und der Prüfeinrichtung (11) angeordneten Reibfläche (9).

9. Vorrichtung nach Anspruch 7 oder 8, **dadurch gekennzeichnet**, daß die Fördervorrichtung (4) mit einer in bestimmbarem Zeittakt schaltbaren Bremsvorrichtung ausgestattet ist, die ermöglicht, daß die Drehbewegung der Fördervorrichtung intermittierend erfolgt und dann zu unterbrechen ist, wenn ein Prüfling (2a, b...n) nach erfolgter Drehung um seine eigene Längsachse in den Bereich einer der außerhalb des Umfangs der Fördervorrichtung (4) stationär angeordneten Prüfeinrichtungen (11) gelangt ist.

10. Vorrichtung nach einem der Ansprüche 7 bis 9, **dadurch gekennzeichnet,** daß die Reibflächen (9) durch einen Belag aus einem gummielastischem Material (Schlauch) besteht, der längs der Innenfläche eines die Fördervorrichtung (4) längs ihres Umfangsbereiches umgebenen Außenrandes (7) angeordnet ist.

11. Vorrichtung nach einem der Ansprüche 7 bis 10, **dadurch gekennzeichnet,** daß der Außenrand (7) der Fördervorrichtung (4) in einem Abstand voneinander längs des Außenrandes angeordnete Führungsscheiben (22a,b) aufweist, wobei der Abstand der Führungsscheiben nach Maßgabe der Flüssigkeitssäule und/oder der Behälterform der Prüflinge gewählt ist.

12. Vorrichtung nach einem der Ansprüche 7 bis 11, **dadurch gekennzeichnet**, daß die Rollen- oder Gleitlager der Fördervorrichtung (4) als Doppelrollen (20a,b, 21a,b) bzw. Doppelgleitlager mit übereinstimmender Drehachse ausgebildet sind, wobei die Rollen- bzw. Gleitlager übereinander in einem durch die Längsausdehnung der Prüflinge (19) bestimmten Abstand angeordnet sind.

13. Vorrichtung nach einem der Ansprüche 7 bis 12, **dadurch gekennzeichnet**, daß die Reibvorrichtung einen Förderkanal (27) umfaßt, dessen eine Längsseitenwand durch die Reibfläche (30) gebildet ist, deren Reibungskoeffizient groß in Bezug auf das Material der Prüflinge ist und durch die die Prüflinge bei ihrer Bewegung durch den Förderkanal zur Rotation gebracht werden und daß die Fördervorrichtung einen hin- und herbewegbaren Stössel (31) aufweist, der die Prüflinge zumindest bis zum Förderkanal (27) transportiert und daß die Prüflinge nach Durchlaufen des Förderkanales (27) in Ruhepostion im Bereich der Prüfeinrichtung (32) beobachtbar und beurteilbar sind, bevor sie zur Ausgabevorrichtung gelangen.

14. Vorrichtung nach Anspruch 13, **dadurch gekennzeichnet,** daß die Fördervorrichtung zum Transport der Prüflinge durch den Kanal (27) ein umlaufendes Transportband (28) umfaßt, das für die Führung der Prüflinge mit Rollen (29) oder dergleichen versehen ist.

15. Vorrichtung nach Anspruch 13 oder 14, **dadurch gekennzeichnet,** daß der hin- und herbewegbare Stössel (31) zur Beförderung der Prüflinge durch den Kanal (27) dient und an seiner Stirnfläche mit einer oder mehreren Rollen (36) versehen ist und daß die Reibfläche (30) in Form eines vorzugsweise umlaufenden Reibbandes (37) ausgebildet ist.

16. Vorrichtung nach einem der Ansprüche 7 bis 15, **dadurch gekennzeichnet**, daß die Fördervorrichtung als eine Förderschnecke (42) ausgebildet ist, die dazu dient, die aus der Zuführungsvorrichtung (40) aufgenommenen Prüflinge (2a..n) längs der oder den Reibflächen (43, 44) zur Prüfeinrichtung (45) hin zu bewegen.

17. Vorrichtung nach Anspruch 16, **dadurch gekennzeichnet**, daß die Reibflächen als zwei sich parallel zueinander und zur Längsachse der Förderschnecke (42) erstreckende, gegensinnig umlaufende Reibbänder (43,44) ausgebildet sind, deren Umlaufgeschwindigkeiten so gewählt sind, daß ihre Differenz der Fördergeschwindigkeit der Förderschnecke in Höhe und Richtung entspricht.

18. Vorrichtung nach Anspruch 16 oder 17, **dadurch gekennzeichnet**, daß für die Rotation ein Elektromotor und für die Hin- und Herbewegung der Förderschnecke (42) Federn und Nocken (nicht dargestellt) vorgesehen sind.

19. Vorrichtung nach Anspruch 18 , **dadurch gekennzeichnet,** daß die Amplitude der Hin- und Herbewegung der Förderschnecke (42) dem Abstand zwischen der Stirnfläche der Förderschnecke und der Prüfposition (49) der Prüflinge (2a..n) in der Prüfeinrichtung (45) entspricht.

20. Vorrichtung nach einem der Ansprüche 16 bis 19, **dadurch gekennzeichnet**, daß in der Prüfeinrichtung (45) eine Bremsvorrichtung (50) vorgesehen ist, die dazu dient, den von der Förderschnecke (42) angelieferten Prüfling (2a..n) vor Beginn der Prüfung zum Stillstand zu bringen.
